# FASCICULE DE BREVET EUROPEEN

(11) **EP 0 618 184 B1**
(45) Date de publication et mention de la délivrance du brevet: **14.01.1998**
(21) Numéro de dépôt: 94400678.2
(22) Date de dépôt: 30.03.1994
(51) Int. Cl.: C07C 51/12, C07C 67/36, C07C 67/37

(54) **Procédé de préparation d'acides carboxyliques ou des esters correspondants en présence d'un catalyseur à base d'iridium**
Verfahren zur Herstellung von Carbonsäuren oder ihrer Ester in gegenwart des auf Iridium basierten Katalysators
Process for the preparation of carboxylic acids or corresponding esters in presence of a catalyst based on iridium

(30) Priorité: 31.03.1993 FR 9303734; 10.12.1993 FR 9314844; 29.12.1993 FR 9315825
(43) Date de publication de la demande: 05.10.1994
(73) Titulaire: PARDIES ACETIQUES, 92082 Paris La Défense 2 (FR)
(72) Inventeur: Nobel, Dominique, F-69270 Fontaines/Saint/Martin (FR); Perron, Robert, F-69390 Charly (FR); Denis, Philippe, F-69150 Decines (FR)
(74) Mandataire: Portal, Gérard

(56) Documents cités:
- FR-A- 2 317 269
- US-A- 3 772 380

## Description

La présente invention concerne un procédé de préparation d'acides carboxyliques ou des esters correspondants, par carbonylation, en phase liquide, d'un alcool, en présence d'un catalyseur à base d'iridium.

L'obtention d'acides carboxyliques, et plus particulièrement d'acide acétique, par réaction de monoxyde de carbone avec un alcool comme le méthanol, en présence d'un catalyseur homogène est un procédé bien connu, ayant fait l'objet de nombreux brevets et articles. Les catalyseurs susceptibles d'être utilisés dans ce type de réaction sont entre autres le cobalt, le rhodium et l'iridium.

Seuls les procédés de carbonylation utilisant des catalyseurs à base de rhodium sont exploités actuellement à une échelle industrielle et font l'objet de nouveaux développements. Les dernières générations de ces procédés mettent en oeuvre le rhodium, des quantités importantes de sels solubles d'iodures stabilisant le métal précité et de faibles teneurs en eau. Ils sont très performants puisqu'ils permettent d'atteindre des vitesses de carbonylation du méthanol en acide acétique supérieures à 10 mol/l.h.

La préparation d'acides carboxyliques catalysée par le cobalt n'est plus retenue actuellement pour faire l'objet de nouvelles exploitations du fait des conditions de réaction très dures de pressions et de températures pour obtenir des résultats, en terme de sélectivités en acide formé, relativement peu satisfaisantes, en comparaison des contraintes à gérer.

Quant aux procédés utilisant une catalyse à base d'iridium, les résultats mis en évidence sont très peu performants. En effet, les vitesses de carbonylation rapportées sont de l'ordre de 2 à 4 mol/h.l d'acide formé alors que le nombre de moles engagé dans la réaction est très important. Ceci correspond à une activité du catalyseur, rapportée au nombre de moles d'acide acétique produit par mole d'iridium et par heure, inférieure à 1000.

La présente invention a donc pour but de proposer un mode de préparation d'acides carboxyliques par une réaction de carbonylation d'un réactif approprié, ne mettant pas en oeuvre un catalyseur à base de rhodium, tout en conservant une productivité comparable à celles des procédés utilisant le catalyseur précité.

Ainsi, le procédé selon l'invention a pour objet un procédé de préparation en continu d'acides carboxyliques ou des esters correspondants présentant (n+1) atomes de carbone, par réaction en phase liquide, de monoxyde de carbone avec au moins un alcool présentant n atomes de carbone, en présence d'un système catalytique à base d'un composé de l'iridium et d'un promoteur halogéné, selon lequel on maintient dans le milieu, pendant la réaction, l'eau, dans une teneur variant entre 0 exclus et 10 %, le promoteur halogéné dans une teneur variant entre 0 exclus et 10%, l'ester correspondant à l'acide carboxylique et à l'alcool précité, dans une teneur variant entre 2 et 40 %, et l'alcool entre O et 10%, de préférence entre 0, 1 et 8 %, ledit acide carboxylique précité constituant le solvant de la réaction.

Dans tout ce qui va suivre, et sauf indication contraire, les pourcentages indiqués sont exprimés en poids, rapportés au poids total du mélange réactionnel.

Il a été trouvé de façon totalement surprenante que le procédé selon l'invention, réalisé en présence d'un catalyseur à base d'iridium dans les conditions stables explicitées ci-dessus, est beaucoup plus performant que les procédés décrits mettant en oeuvre un tel catalyseur. De plus, et ce fait est important, le procédé selon l'invention permet d'atteindre des vitesses de carbonylation de l'alcool en acide carboxylique, exprimées en moles, comparables à celles obtenues avec les procédés catalysés par le rhodium, en mettant en oeuvre des quantités de catalyseur similaires.

Par ailleurs, on a constaté, contrairement à ce que l'on aurait pu attendre, que le catalyseur restait stable dans un milieu réactionnel présentant une faible teneur en eau, même en l'absence de tout type de composé connu stabilisant le catalyseur, qu'il soit de type organique ou minéral. Par conséquent, le procédé selon l'invention présente l'avantage de ne pas nécessiter l'emploi de tels composés pendant la réaction.

Il est de même à noter que le procédé selon l'invention est mis en oeuvre en présence de quantités relativement faibles de promoteur halogéné. Ceci présente les avantages de diminuer la quantité de promoteur à séparer de l'acide formé et de diminuer la consommation d'énergie nécessaire à la récupération de ce composé halogéné ainsi que la consommation spécifique en ce composé, c'est-à-dire sa consommation lors d'une exploitation en continu du procédé de carbonylation. Par ailleurs, cette mesure a permis de baisser, pour des teneurs en eau et en ester données, la teneur en hydracide correspondant à l'halogène du promoteur dans le milieu. Par conséquent la corrosivité dudit milieu est diminuée, rendant plus facile et moins coûteux le choix des matériaux, mis en contact avec un tel milieu.

On a enfin constaté que l'iridium mis en oeuvre dans de telles conditions était remarquablement sélectif, en ce sens que la quantité de sous-produits formés, comme par exemple l'acide propionique, l'acide formique, est très faible.

En effet, la quantité des deux acides précités formés pendant la réaction est en général, pour chacun d'eux, inférieure à 50 mg/kg d'acide ou d'ester produit.

Mais d'autres buts et avantages de la présente invention apparaîtront plus clairement à la lecture de la description qui va suivre.

Ainsi qu'il a été dit précédemment, la réaction de carbonylation de l'invention est réalisée en présence d'un système catalytique à base d'au moins un composé de l'iridium et d'un promoteur halogéné.

La réaction étant effectuée en phase liquide, le système catalytique se présente sous forme de composés solubles dans le mélange réactionnel.

Tous les composés de l'iridium solubles ou pouvant être solubilisés dans le milieu réactionnel, dans les conditions de réalisation de l'invention, peuvent être utilisés. A titre d'exemple et sans intention de se limiter, conviennent notamment à la mise en oeuvre de l'invention, l'iridium à l'état métallique, les sels simples de ce métal, les oxydes ou encore les complexes de coordination.

En tant que sels simples d'iridium, on utilise habituellement les halogénures d'iridium. L'halogène est plus particulièrement choisi parmi le chlore, le brome ou l'iode, ce dernier étant préféré. Ainsi les composés comme lrl₃, lrBr₃, lrCl₃, lrl₃.4H₂O, lrBr₃.4H₂O peuvent être utilisés dans le procédé selon l'invention.

Les oxydes choisis parmi lrO₂, lr₂O₃.xH₂O peuvent de même être convenablement mis en oeuvre dans le procédé selon l'invention.

En ce qui concerne les complexes solubles de coordination de l'iridium, les composés les plus couramment mis en oeuvre sont ceux présentant des ligands choisis parmi le monoxyde de carbone ou une combinaison monoxyde de carbone/halogène, l'halogène étant choisi parmi le chlore, le brome ou plus particulièrement l'iode. Il n'est toutefois pas exclus d'utiliser des complexes solubles d'iridium dont les ligands sont choisis parmi des composés organo phosphorés ou organo azotés, par exemple.

En tant que complexes de coordination, connus de l'homme du métier, convenant particulièrement à la mise en oeuvre de l'invention, on peut citer sans intention de se limiter les composés suivants : lr₄(CO)₁₂, lr(CO)₂l₂⁻Q⁺, lr(CO)₂Br₂⁻Q⁺, lr(CO)₂Cl₂⁻Q⁺ ; formules dans lesquelles Q peut représenter notamment l'hydrogène, le groupe NR₄, PR₄, avec R choisi parmi l'hydrogène, un radical hydrocarboné.

Ces catalyseurs peuvent être obtenus par toute méthode connue de l'homme du métier.

Cependant, selon un mode particulièrement avantageux, on peut préparer une solution catalytique à partir d'un composé carbonylé d'iridium, tel que lr₄CO₁₂, par mise en contact dudit composé avec de l'acide iodhydrique et/ou un précurseur d'un tel acide, en présence d'un solvant. Comme précurseur susceptible de libérer l'acide iodhydrique, on peut mentionner à titre d'exemple, l'iode, les iodures d'alkyle en C₁-C₁₀, ou encore les iodures alcoyles en C₁-C₁₀. En ce qui concerne les solvants, tous les composés peuvent être utilisés dans la mesure où ils solubilisent l'acide iodhydrique ou son précurseur et le composé à base d'iridium obtenu. Plus particulièrement on met en oeuvre des solvants, seuls ou en mélange, choisis parmi l'eau, les acides carboxyliques ou les esters correspondants, obtenus par le procédé selon l'invention. La mise en contact à lieu sous une pression totale comprise entre 1 et 10 bar et à une température au plus égale à la température d'ébullition du solvant précité, dans les conditions de la mise en contact. L'opération peut être effectuée sous air, sous un gaz inerte ou encore sous monoxyde de carbone.

Un autre exemple de méthode avantageuse pour la préparation d'une solution catalytique convenable à la mise en oeuvre de l'invention, consiste à mettre en contact, en phase liquide, un ou plusieurs oxydes hydratés ou non d'iridium, avec de l'acide iodhydrique ou un composé susceptible de libérer de l'acide iodhydrique. L'acide iodhydrique peut être employé sous la forme d'un gaz, d'une solution, plus particulièrement aqueuse. Il peut encore être utilisé sous la forme d'un précurseur, notamment comme ceux mentionnés dans la précédente variante. Plus particulièrement la quantité d'acide iodhydrique est telle que le rapport entre le nombre de moles d'acide iodhydrique au nombre de moles d'iridium, varie entre 1 et 100. Le procédé selon l'invention peut être mis en oeuvre sous air, sous un gaz inerte, sous monoxyde de carbone, ces gaz étant seuls ou en combinaison.

Généralement, la concentration totale en iridium dans le milieu réactionnel est comprise entre 0,1 et 100 mmol/l de préférence entre 1 et 25 mmol/l.

Le second constituant du système catalytique est un promoteur halogéné. Celui-ci peut se présenter sous la forme d'un halogène seul, ou en combinaison avec d'autres éléments comme par exemple l'hydrogène, un radical alkyle en C₁-C₁₀, un radical acyle en C₁-C₁₀, un radical aryle en C₆-C₁₀.

L'halogène est en général choisi parmi le chlore, le brome ou l'iode, ce dernier étant préféré.

A titre d'exemple de composés halogénés convenant à la mise en oeuvre de l'invention, on peut citer entre autres l'iode, l'acide iodhydrique, l'acide bromhydrique, l'iodure de méthyle, le bromure de méthyle, l'iodure d'éthyle, le bromure d'éthyle, le di iodure-1,1 d'éthyle, le bromure de benzyle, l'iodure d'acétyle.

Selon un mode de réalisation particulier de l'invention, le promoteur mis en oeuvre comprend l'hydrogène ou un radical alkyle en C₁-C₁₀. Plus particulièrement, le promoteur utilisé dans l'invention comprend l'halogène et un radical alkyle en C₁-C₁₀.

De préférence, ce mode de réalisation est effectué en présence d'un promoteur halogéné dont le radical correspond à celui de l'alcool utilisé comme réactif lors de la réaction selon l'invention.

La teneur en promoteur halogéné dans le milieu est comprise entre 0 exclus et 10 %. Selon un mode particulier de réalisation de l'invention, la teneur en composé halogéné dans le milieu réactionnel est comprise entre 1 et 6 %.

Les réactifs mis en oeuvre dans le procédé selon l'invention vont maintenant être présentés.

Comme cela a été mentionné auparavant, la réaction selon l'invention est effectuée en présence d'un alcool comprenant un atome de carbone en moins par rapport à l'acide carboxylique ou à l'ester correspondant, fabriqué.

Parmi les réactifs convenables à la mise en oeuvre de la réaction, on peut citer les alcools, saturés, présentant un à dix atomes de carbone. Les alcools peuvent être mono ou dihydroxylés. A titre d'exemples de tels composés, on peut citer notamment le méthanol, l'éthanol, le propanol, le butanol, le 1,4-butanediol.

Selon un mode de réalisation préféré, les alcools utilisés sont choisis parmi les composés monohydroxylés.

Il est important de noter que l'alcool utilisé comme réactif peut être présent dans le milieu réactionnel en tant que tel ou sous forme masquée. En effet, ledit alcool peut se trouver indifféremment sous la forme d'un dérivé halogéné et/ou d'un éther et/ou d'un ester obtenu par réaction entre ledit alcool et l'acide carboxylique présent.

Ainsi, la teneur en réactif dans le milieu réactionnel peut varier dans de larges limites, du fait des différentes espèces sous lesquelles le réactif peut se présenter.

Par conséquent, la teneur en alcool en tant que tel, dans le milieu réactionnel peut être comprise entre 0 et 10 %. De préférence, le milieu comprend une teneur en alcool comprise entre 0,1 et 8 %.

L'autre réactif nécessaire à l'obtention d'un acide carboxylique est le monoxyde de carbone. Celui-ci peut être utilisé sous forme pure ou diluée dans des gaz tels que l'hydrogène, le méthane, le dioxyde de carbone, ou tout autre type de gaz comme par exemple l'azote.

Selon un mode particulier de réalisation de l'invention, on utilise un monoxyde de carbone présentant une pureté d'au moins 99 %.

La pression partielle en monoxyde de carbone est habituellement comprise entre 10 et 50 bar et de préférence entre 10 et 20 bar, bien que des pressions partielles en monoxyde de carbone en dehors de ces gammes soient envisageables.

La réaction de carbonylation selon l'invention est effectuée en outre, en présence d'eau, la teneur en eau dans le milieu réactionnel étant comprise entre 0 exclus et 10 %. Selon un mode de réalisation particulier de l'invention, la teneur en eau dans le milieu est comprise entre 2 et 8 %.

Outre les composés et réactifs précédemment mentionnés, le procédé selon l'invention est réalisé en présence d'esters correspondant, de préférence, à la réaction de l'alcool mis en jeu dans la réaction, avec l'acide carboxylique présent dans le milieu réactionnel. Plus particulièrement, la teneur en ester dans ledit milieu est comprise entre 2 et 40%. Selon un mode de réalisation particulier de l'invention, la teneur en ester est comprise entre 5 et 30%.

Enfin, le procédé selon l'invention est effectué dans un solvant qui, de façon avantageuse, correspond à l'acide carboxylique ou l'ester formé par la réaction.

Ainsi que cela a été indiqué auparavant, la présente invention consiste à maintenir dans le milieu réactionnel, l'eau, le promoteur halogéné, l'ester précité et l'acide carboxylique, dans les proportions venant d'être explicitées. Par conséquent, la présente invention est principalement destinée à être mise en oeuvre en continu et les conditions stables de fonctionnement du procédé correspondent à la composition et aux proportions indiquées.

Lors du démarrage de la réaction, les divers composants sont introduits dans un réacteur approprié, muni de moyens d'agitation suffisants pour assurer le transfert gaz-liquide. Il est à noter que si le réacteur comprend de préférence des moyens d'agitation mécanique du mélange réactionnel, il n'est pas exclus d'opérer sans de tels moyens, l'homogénéisation du mélange pouvant être réalisée par l'introduction du monoxyde de carbone dans le réacteur.

Les composants du milieu réactionnel sont introduits sans ordre préférentiel, sous leur forme propre et/ou sous la forme d'un ou plusieurs précurseurs.

Une première variante de l'invention consiste à introduire le promoteur halogéné décrit auparavant tel quel dans le mélange réactionnel.

Une seconde variante de mise en oeuvre, consiste à introduire ledit promoteur sous la forme d'au moins un précurseur.

Dans ce cas de figure, ce précurseur se présente généralement sous la forme d'un composé susceptible de libérer dans le milieu réactionnel le radical précité du promoteur halogéné précité, par réaction dudit précurseur avec un halogène ou l'hydracide correspondant, présent dans le milieu ou bien introduit à cet effet.

A titre d'exemple non limitatif de précurseurs convenables, on peut citer les composés choisis parmi les alcools de formule (1) ROH ; les éthers de formule (2) ROR' ou encore des esters de formules (3) R'COOR, utilisés seuls ou en mélange. Dans les formules précitées, les radicaux R et R', identiques ou différents, représentent chacun un radical alkyle en C₁-C₁₀, acyle en C₁-C₁₀, ou aryle en C₆-C₁₀ ; avec le radical R correspondant au radical du promoteur halogéné.

Ainsi, le méthanol, I'éthanol, le propanol, le butanol, le diméthyl éther, le diéthyl éther, l'oxyde d'éthylène, l'acétate de méthyle, sont des précurseurs convenables dudit promoteur halogéné.

Habituellement la réaction de carbonylation est réalisée à une température comprise entre 150 et 250 °C. De préférence la température de réaction varie entre 180 et 210°C.

La pression totale est généralement comprise entre 5 et 200 bar et plus particulièrement entre 5 et 100 bar.

Il est à noter que le procédé selon l'invention peut être convenablement mis en oeuvre dans les installations exploitant les procédés classiques. Ainsi, ces procédés peuvent être séparés principalement en trois zones. La première correspond à la zone de réaction, comprenant un réacteur sous pression ; la seconde est celle de séparation de l'acide, ou de l'ester formé, par vaporisation partielle du mélange réactionnel. La partie vaporisée est ensuite envoyée dans une zone de purification de l'acide carboxylique ou de l'ester correspondant; la partie du mélange restée sous forme liquide, comprenant principalement le catalyseur, est recyclée au réacteur.

Selon un mode de réalisation particulier du procédé selon l'invention, le mélange réactionnel est purgé régulièrement des métaux de corrosion qu'il contient, dont notamment le fer, le molybdène, le chrome, le nickel. Cette opération est réalisée selon tout moyen connu de l'homme du métier, comme par exemple le traitement du mélange réactionnel par une résine échangeuse d'ions ou encore par précipitation du catalyseur et séparation de ce dernier, des métaux de corrosion, par filtration.

Le procédé selon l'invention convient à la fabrication de tout type d'acide carboxylique ou des esters correspondants, comprenant au minimum deux atomes de carbone. Ainsi, celui-ci peut être mis en oeuvre pour préparer l'acide propionique à partir de l'éthanol, l'acide succinique à partir de l'oxyde d'éthylène, l'acide adipique à partir du 1,4-butanediol, ou les esters correspondants.

Cependant, ce procédé convient tout particulièrement à l'obtention d'acide acétique et/ou d'acétate de méthyle à partir de méthanol.

Selon un mode de réalisation préféré de l'invention, le procédé selon l'invention est mis en oeuvre à partir d'iodure de méthyle, d'acétate de méthyle et d'acide acétique en tant que solvant, outre le méthanol.

Des exemples concrets mais non limitatifs de l'invention vont maintenant être présentés.

### EXEMPLE 1

Les essais suivants ont été réalisés en continu dans un autoclave de 300 cm³ muni d'un moyen d'agitation mécanique et de moyens d'introduction des réactifs.

Le milieu réactionnel comprend du chlorure d'iridium avec une concentration de 3 mmol/l, dans l'acide acétique.

Les introductions de méthanol, d'iodure de méthyle et d'eau sont réglées de telle sorte que les teneurs des différents composants dans le milieu réactionnel soient maintenues comme indiquées dans le tableau ci-dessous.

Le temps de séjour dans le réacteur est d'environ 10 minutes.

La pression totale dans l'autoclave est de 30 bar et la température est maintenue à 190°C.

A la sortie de l'autoclave, le mélange réactionnel est dégazé et refroidi.

Le mélange et les gaz sont analysés par chromatographie en phase gazeuse.

**tableau 1**

| **ESSAIS** | **H**_{**2**}**O** | **CH**_{**3**}**l** | **CH**_{**3**}**CO**_{**2**}**H** | **CH**_{**3**}**CO**_{**2**}**CH**_{**3**} | **V**_{**CARB**} | **ACTIVITE** |
|---|---|---|---|---|---|---|
| **A** | 6,6 | 9,7 | 56 | 26 | 11,2 | 4600 |
| **B** | 6,7 | 7,7 | 60 | 26 | 11 | 4700 |
| **C** | 6,9 | 5,2 | 61 | 27 | 10,6 | 4800 |
| **D** | 6,8 | 3,6 | 61 | 27 | 9,3 | 4600 |

V_{CARB} représente la vitesse de carbonylation, exprimée en mol/l.h.

Elle est obtenue par mesure du débit de consommation du CO en tenant compte, en outre, de la quantité de ce gaz engagée dans la formation de CO₂.

Les activités correspondent au nombre de moles d'acide acétique formé, par moles d'iridium par heure et par litre.

Les teneurs des différents constituants du mélange sont exprimées en % poids, rapporté au poids total du mélange réactionnel.

Ces essais montrent que la vitesse de carbonylation est considérablement améliorée par rapport à celles indiquées dans l'art antérieur.

### EXEMPLE 2

On opère selon le mode opératoire décrit dans l'exemple 1 mais les compositions du mélange réactionnel sont les suivantes :

**tableau 2**

| **ESSAIS** | **H**_{**2**}**O** | **CH**_{**3**}**l** | **CH**_{**3**}**CO**_{**2**}**H** | **CH**_{**3**}**CO**_{**2**}**CH**_{**3**} | **V**_{**CARB**} | **ACTIVITE** |
|---|---|---|---|---|---|---|
| **A** | 3,0 | 8,9 | 87 | 12 | 6,5 | 2500 |
| **B** | 3,1 | 6,1 | 80 | 12 | 5,9 | 2400 |
| **C** | 3,2 | 4,0 | 80 | 12 | 5,6 | 2300 |

Les unités des différentes valeurs dans ce tableau sont identiques à celles indiquées dans le tableau 1.

Ces essais montrent que la vitesse de carbonylation reste élevée par rapport aux procédés décrits mettant en oeuvre l'iridium, même avec de faibles teneurs en eau, sans constater, par ailleurs, de précipitation du catalyseur.

### EXEMPLE 3

On opère de la même façon que dans les exemples précédents.

Les introductions d'eau, d'iodure de méthyle et de méthanol et d'acide acétique sont réglées pour maintenir les compositions ci-dessous :

**tableau 3**

| **ESSAIS** | **H**_{**2**}**O** | **CH**_{**3**}**OH** | **CH**_{**3**}**CO**_{**2**}**CH**_{**3**} | **CH**_{**3**}**CO**_{**2**}**H** | **CH**_{**3**}**l** | **lr** | **V**_{**CARB**} | **ACTIVITE** |
|---|---|---|---|---|---|---|---|---|
| **A** | 7,3 | 1,3 | 27 | 56 | 9,8 | 2,9 | 13 | 4400 |
| **B** | 5,1 | 0,56 | 20 | 71 | 3,8 | 2,2 | 8,6 | 3900 |
| **C** | 5,9 | 0,77 | 23 | 65 | 5,6 | 2,3 | 10,1 | 4500 |
| **D** | 6,3 | 1,1 | 24,9 | 57,6 | 7,7 | 2,3 | 11,4 | 4900 |

Les teneurs en iridium sont exprimées en mmol/l.

Les autres valeurs sont exprimées dans les mêmes unités que celles du tableau 1.

La teneur en HI dans le milieu est comprise entre 4 et 7 mmol/l.

### EXEMPLE 4

On procède comme pour l'exemple 1, excepté le fait que le catalyseur utilisé est obtenu à partir de lr₄CO₁₂ et est préparé comme suit :

On introduit dans un ballon en verre, 10 g de lr₄CO₁₂, 50 g d'acide iodhydrique en solution à 57 % dans l'eau et 290 g d'acide acétique.

Le mélange est chauffé à reflux sous agitation et sous air pendant 4 heures.

On effectue la réaction de carbonylation en continu, en présence 1,9 mmol/l d'iridium provenant de la solution obtenue précédemment, en maintenant une composition du mélange réactionnel correspondant à:
4 % d'eau,
3,3 % d'iodure de méthyle,
28 % d'acétate de méthyle,
le complément étant de l'acide acétique.

On obtient dans de telles conditions une vitesse de carbonylation de 4,3 mol/h.l, correspondant à une activité de 2300 moles d'acide formé par mole d'iridium, par heure et par litre.

La quantité d'acide propionique produite est inférieure à 23 mg/kg d'acide acétique produit.

La quantité d'acide formique produite est inférieure à 25 mg/kg d'acide acétique produit.

### EXEMPLE 5

On réalise la carbonylation en présence d'un catalyseur obtenu à partir de Ir₄CO₁₂ obtenu selon le même mode opératoire que dans l'exemple précédent, en maintenant la composition réactionnelle suivante (% exprimés en poids) :
1,8 mmol/l d'iridium,
9,9 % d'eau,
3,6 % d'iodure de méthyle,
18 % d'acétate de méthyle,
le complément étant l'acide acétique.

On obtient une vitesse de carbonylation de 5,4 mol/h.l, correspondant à une activité de 3000 moles d'acide formé par mole d'iridium, par heure et par litre.

Les quantités d'acides propionique et formique formées sont chacune inférieures à 25 mg/kg d'acide acétique produit.

## Revendications

1. Procédé de préparation en continu d'acides carboxyliques ou des esters correspondants présentant (n+1) atomes de carbone, par réaction en phase liquide, de monoxyde de carbone avec au moins un alcool présentant n atomes de carbone. en présence d'un système catalytique à base d'un composé de l'iridium et d'un promoteur halogéné, caractérisé en ce que l'on maintient dans le milieu, pendant la réaction, l'eau, dans une teneur variant entre 0 exclus et 10 %, le promoteur halogéné, dans une teneur variant entre 0 exclus et 10%, l'ester correspondant à l'acide carboxylique et à l'alcool précités, dans une teneur variant entre 2 et 40 %, et l'alcool entre 0 et 10 %, de préférence entre 0,1 et 8 %,ledit acide carboxylique constituant le solvant de la réaction.

2. Procédé selon la revendication précédente, caractérisé en ce que l'on maintient dans le mélange une teneur en eau comprise entre 2 et 8 %.

3. Procédé selon l'une des revendications précédentes, caractérisé en ce que l'on maintient dans le mélange une teneur en promoteur halogéné comprise entre 1 et 6 %.

4. Procédé selon l'une des revendications précédentes, caractérisé en ce que l'on maintient dans le mélange une teneur en ester précité comprise entre 5 et 30%.

5. Procédé selon l'une des revendications précédentes, caractérisé en ce que l'on utilise un alcool hydrocarboné saturé en C₁-C₁₀.

6. Procédé selon l'une des revendications précédentes, caractérisé en ce que l'on utilise un promoteur halogéné comprenant un halogène choisi parmi le chlore le brome ou l'iode, en combinaison avec de l'hydrogène, un radical alkyle en C₁-C₁₀, un radical acyle en C₁-C₁₀ ou un radical aryle en C₆-C₁₀.

7. Procédé selon la revendication précédente, caractérisé en ce que l'on utilise un promoteur halogéné dont le radical correspond à celui de l'alcool précité.

8. Procédé selon l'une des revendications précédentes, caractérisé en ce que l'on prépare l'acide acétique par réaction du méthanol avec du monoxyde de carbone, en présence d'eau, d'iodure de méthyle, d'acétate de méthyle, l'acide acétique constituant le solvant de la réaction.

## Patentansprüche

1. Verfahren zur kontinuierlichen Herstellung von Carbonsäuren oder entsprechenden Estern, die (n+1) Kohlenstoffatome aufweisen, durch Umsetzung in flüssiger Phase von Kohlenmonoxid mit mindestens einem Alkohol, der n-Kohlenstoffatome aufweist, in Gegenwart eines katalytischen Systems auf Basis einer Iridiumverbindung und eines halogenierten Promotors, dadurch gekennzeichnet, daß man während der Umsetzung in dem Medium das Wasser bei einem Gehalt hält, der zwischen 0 (unter Ausschluß des Wertes 0) und 10 % variiert, den halogenierten Promotor bei einem Gehalt hält, der zwischen 0 (unter Ausschluß des Wertes 0) und 10 % variiert, den der obengenannten Carbonsäure und dem obengenannten Alkohol entsprechenden Ester bei einem Gehalt hält, der zwischen 2 und 40 % variiert, und den Alkohol bei einem Gehalt hält, der zwischen 0 und 10 %, vorzugsweise zwischen 0,1 und 8 %, variiert, wobei die genannte Carbonsäure das Lösungsmittel für die Umsetzung darstellt.

2. Verfahren nach dem vorhergehenden Anspruch, dadurch gekennzeichnet, daß man in dem Gemisch einen Wassergehalt zwischen 2 und 8 % aufrechterhält.

3. Verfahren nach einem der vorhergehenden Ansprüche, dadurch gekennzeichnet, daß man in dem Gemisch einen Gehalt an halogeniertem Promotor zwischen 1 und 6 % aufrechterhält.

4. Verfahren nach einem der vorhergehenden Ansprüche, dadurch gekennzeichnet, daß man in dem Gemisch einen Gehalt an dem obengenannten Ester zwischen 5 und 30 % aufrechterhält.

5. Verfahren nach einem der vorhergehenden Ansprüche, dadurch gekennzeichnet, daß man einen gesättigten C₁-C₁₀-Kohlenwasserstoffalkohol verwendet.

6. Verfahren nach einem der vorhergehenden Ansprüche, dadurch gekennzeichnet, daß man einen halogenierten Promotor verwendet, der ein Halogen, ausgewählt aus der Gruppe Chlor, Brom oder lod, in Kombination mit Wasserstoff, einen C₁-C₁₀-Alkylrest, einen C₁-C₁₀-Acylrest oder einen C₆-C₁₀-Arylrest enthält.

7. Verfahren nach dem vorhergehenden Anspruch, dadurch gekennzeichnet, daß man einen halogenierten Promotor verwendet, dessen Rest demjenigen des vorgenannten Alkohols entspricht.

8. Verfahren nach einem der vorhergehenden Ansprüche, dadurch gekennzeichnet, daß man Essigsäure herstellt durch Umsetzung von Methanol mit Kohlenmonoxid in Gegenwart von Wasser, Methyliodid und Methylacetat, wobei die Essigsäure das Lösungsmittel für die Umsetzung darstellt.

## Claims

1. A continuous process for the preparation of carboxylic acids or corresponding esters having (n+1) carbon atoms, by the reaction in liquid phase of carbon monoxide with at least one alcohol having n carbon atoms, in the presence of an iridium compound-based catalytic system and a halogenated promoter, characterised in that the water is maintained in the medium during the reaction at a content ranging between 0 (excluded) and 10%, the ester corresponding to the above-mentioned carboxylic acid and alcohol at a content ranging between 2 and 40%, and the alcohol between 0 and 10%, preferably between 0.1 and 8%, said carboxylic acid constituting the reaction solvent.

2. The process according to the preceding claim, characterised in that a water content is maintained between 2 and 8% in the mixture.

3. The process according to one of the preceding claims, characterised in that a halogenated promoter content is maintained between 1 and 6% in the mixture.

4. The process according to one of the preceding claims, characterised in that a content of the above-mentioned ester is maintained between 5 and 30% in the mixture.

5. The process according to one of the preceding claims, characterised in that a saturated C₁-C₁₀ hydrocarbon alcohol is used.

6. The process according to one of the preceding claims, characterised in that a halogenated promoter is used which comprises a halogen selected from chlorine, bromine or iodine, in combination with hydrogen, a C₁-C₁₀ alkyl radical, a C₁-C₁₀ acyl radical, or a C₆-C₁₀ aryl radical.

7. The process according to the preceding claim, characterised in that a halogenated promoter is used whose radical corresponds to that of the above-mentioned alcohol.

8. The process according to one of the preceding claims, characterised in that acetic acid is prepared by a reaction of methanol with carbon monoxide in the presence of water, methyl iodide, methyl acetate, acetic acid constituting the reaction solvent.
